Europäisches Patentamt

(19) European Patent Office

Office européen des brevets

(11) Publication number : **0 437 377 A1**

(12) **EUROPEAN PATENT APPLICATION**

(21) Application number : 91300210.1

(22) Date of filing : 11.01.91

(51) Int. Cl.⁵ : **F16L 59/14, A61B 17/36,** **H05B 3/58, F17C 13/00**

(30) Priority : 12.01.90 GB 9000723

(43) Date of publication of application : 17.07.91 Bulletin 91/29

(84) Designated Contracting States : AT BE CH DE DK ES FR GB GR IT LI LU NL SE

(71) Applicant : Fern Developments Limited 7 Springburn Place College Milton North East Kilbride Glasgow G74 5NU Scotland (GB)

(72) Inventor : Mackay, Murdo John Norman Inver Cottage, Kittochside, Near Clarkston Glasgow, Scotland (GB) Inventor : Hossack, James 206 Wedderlea Drive, Cardoland Glasgow, Scotland (GB)

(74) Representative : Murgitroyd, Ian G. et al Murgitroyd and Company Mitchell House 333 Bath Street Glasgow G2 4ER (GB)

(54) **Cryogenic conduit.**

(57) A conduit for transmitting cryogenic fluids (16) is formed from a conductive plastics material (10) which acts as both the wall of the conduit and a heater element. The conduit may be flexible and of any suitable cross-section and may be surrounded by a coaxial jacket (12) to form an annular flow passage.

## CRYOGENIC CONDUIT

This invention relates to a conduit for transmitting cryogenic fluids.

It is well known to use cryoprobes for localised cooling in fields such as surgery and semiconductor manufacture. The cryogenic fluid, typically liquid nitrogen, is stored in a flask and is coupled with the cryoprobe via a flexible conduit.

Known conduits for this purpose include simple flexible tubing, which has severe disadvantages in use as the tubing becomes hard and brittle at low temperatures and attracts condensation.

It is also known (see for example EP-A1-0 225 780) to use a conduit around which an electrical resistance wire is wound helically. A current is passed through this wire, in use, such that cryogenic liquid in contact with the conduit wall is vaporised to form a gaseous barrier as an insulation around the bulk of the cryogen which remains in the liquid state ; this is known as the Leidenfrost effect. Conduits of this type have a number of disadvantages. The use of a helical wire heating element produces temperature variation along the length of the conduit which can cause corresponding variation in the thickness of the gas layer and thus non-uniformity of liquid flow. The element is vulnerable to mechanical damage, for example by the conduit being bent acutely or stood upon. The element also occupies a finite amount of space and its removal would allow the diameter of the assembly to be reduced.

According to the present invention, a conduit for cryogenic fluids is formed from a conductive plastics material acting as both conduit wall and heater element.

Preferably, the conduit is of a flexible conductive plastics material, and is preferably of circular cross-section.

The conduit may be surrounded by a coaxial jacket to form an annular flow passage.

Preferably, the conductive plastic material comprises a plastics matrix containing conductive filler and/or reinforcement.

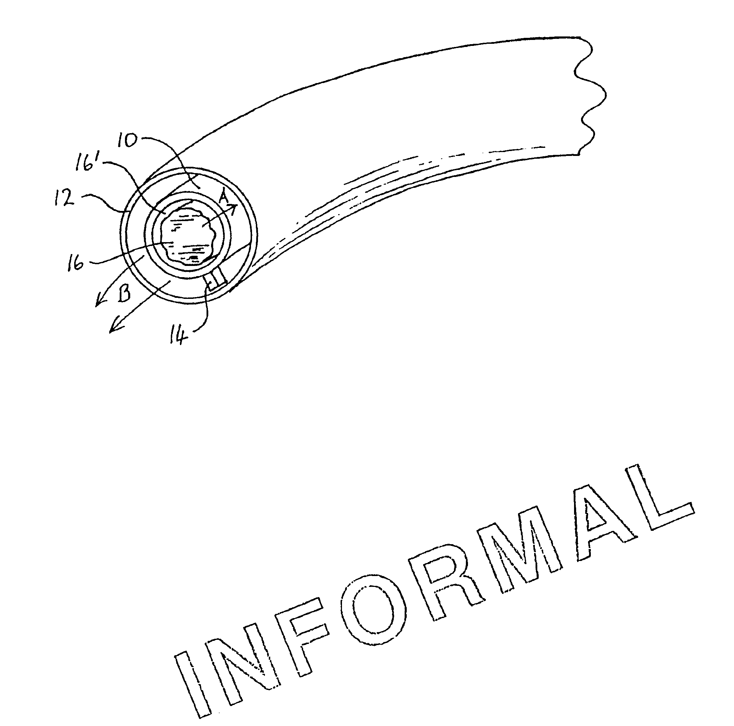

An embodiment of the invention will now be described, by way of example only, with reference to the drawing which is a schematic perspective view, partly in section, of a conduit assembly in accordance with the invention.

The conduit assembly comprises an inner conduit 10 and an outer coaxial jacket 12 which may, for example, be supported by spacers such as 14. A cryogenic liquid 16, typically liquid nitrogen, flows through the conduit 10 in direction A. The conduit 10 and jacket 12 define an annular passage 18 suitable for gas flow such as evaporated cryogen returning from the point of use in direction B.

The conduit 10 is formed of a flexible, electrically conductive plastics material and is connected to a current source (not shown) such that current flow through the material of the conduit 10 produces heating of the conduit. This in turn produces a layer of vaporised cryogen $16^1$ which surrounds and insulates the liquid cryogen 16.

The conductive plastics material comprises a plastics matrix containing electrically conductive filler and/or reinforcement. Suitable reinforcement materials are carbon fibres, stainless steel fibres, aluminium fibres, and metallised glass fibres. Suitable fillers include carbon powder, metal powders and aluminium flakes.

The matrix may be of any suitable plastics material, typically thermoplastics. Various grades of nylon and high density polyethylene are likely to be most useful, but other potential resins include ABS, polycarbonate, acetal, and polysulfones.

The conduit 10 is most conveniently produced as an integral flexible tubular member by extrusion, but the invention may be applied to non-flexible systems, non-circular cross-sections, and built up conduits for example formed by spirally winding strip.

The conductive material may contain additional components such as mechanical reinforcements (eg glass fibre or mica) or lubricants (eg PTFE or silicone)

Modifications and improvements may be incorporated without departing from the scope of the invention.

## Claims

1. A conduit for transmitting cryogenic fluids formed from a conductive plastics material acting as both conduit wall and heater element.

2. A conduit for transmitting cryogenic fluids as claimed in Claim 1, wherein the conduit is of a flexible conductive plastics material.

3. A conduit for transmitting cryogenic fluids as claimed in Claim 1 or Claim 2, wherein the conduit is of circular cross-section.

4. A conduit for transmitting cryogenic fluids as claimed in any one of the preceding Claims, wherein the conduit is surrounded by a coaxial jacket to form an annular flow passage.

5. A conduit for transmitting cryogenic fluids as claimed in any one of the preceding Claims, wherein the plastics material comprises a plastics matrix containing one or more electrically conductive filler.

6. A conduit for transmitting cryogenic fluids as claimed in any one of the preceding Claims, wherein the plastics material comprises a plastics matrix containing electrically conductive reinforcement.

## EUROPEAN SEARCH REPORT

**European Patent Office**

Application Number

EP 91 30 0210

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 225 780 (FERN DEVELOPMENTS LTD) * Claim 1; figures 2,3 * | 1-5 | F 16 L 59/14 A 61 B 17/36 H 05 B 3/58 F 17 C 13/00 |
| Y | FR-A-2 142 784 (VIENNOT & TECALEMIT) * Claim 3 * | 1-5 | |
| P,A | EP-A-0 385 731 (JAPAN GORE-TEX INC.) * Claim 1 * | 1-5 | |

TECHNICAL FIELDS SEARCHED (Int. Cl.5)

F 16 L
A 61 B
H 05 B 3
F 17 C

The present search report has been drawn up for all claims

| Place of search | Date of completion of the search | Examiner |
|---|---|---|
| THE HAGUE | 20-03-1991 | BUDTZ-OLSEN A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)